# EUROPEAN PATENT APPLICATION

(11) **EP 2 359 782 A1**
(43) Date of publication of application: **24.08.2011**
(21) Application number: 10152528.5
(22) Date of filing: 03.02.2010
(51) Int. Cl.: A61F 13/00, A61F 15/00, A61B 17/08, A61F 13/02, A61L 15/42

(54) **Wound closure material**

(71) Applicant: nolax AG, 6203 Sempach Station (CH)
(72) Inventor: Blume, Jessica, 8050, Zürich (CH); Dobmann, Andreas, 6208, Oberkirch (CH); Schwotzer, Willi, 6206, Neuenkirch (CH)
(74) Representative: Hepp Wenger Ryffel AG

(57) **Abstract**

The invention relates to a wound closure material with a core of biodegradable material, wherein at least one side, preferably two sides of the core of biodegradable material are provided with at least one, preferably a multitude of discrete spots of an adhesive. As with more common wound closure techniques like sutures, clips and tapes applied over the wound, new tissue has to close a gap between the wound edges. Use of a wound closing material of the present invention does not only have the advantage of a secure physical connection between the edges, but the material serves as scaffolding for the new tissue. It may also serve the cells as artificial extracellular matrix they can adhere to.

## Description

The present invention relates to a wound closure material with a core of biodegradable material provided on at least one side with at least one, but preferably a multitude of adhesive spots, a method of production of such a wound closure material, use of such a wound closure material and a method to close a wound. Since the closure material is biodegradable, a wound may be closed without the need to remove the material once wound healing is completed.

Small wounds may simply be protected by applying a wound cover such as an adhesive plaster or gauze on top of the wound. Closure of the wound will then occur by natural wound closing mechanisms. Bigger wounds stemming from injuries or from surgical interventions have to be closed and the wound edges physically held together. Traditionally this is being done by classical surgical sutures. Another possibility is to use suture clips. Sutures and clips may both be made from biodegradable material, thus eliminating the need of removing them after completion of wound healing. Another alternative is the use of a strong adhesive tape applied over the wound to hold the edges of the wound together.

Throughout the history of medicine, different adhesive materials were tested as wound closure material. Early attempts used herbal adhesives, such as resins, or mixtures of different animal, plant and mineral material to glue the wound edges together.

Today, even though adhesives are of increasing importance in most fields of technology, such as transportation, construction, etc., there are only very few adhesives used in medicine. Almost 90% of all medicinal adhesives are used in topical applications in the form of patches, wound dressings or as structural dental adhesives and fillings.

Topical tissues, by definition, are exposed to the outside world and are 'dry' so that their moisture content correlates with the ambient humidity. The cells of topical tissues often are 'dead' in the sense that they no longer have a metabolism. Adhesives for topical applications need to have adhesive strengths to match their respective purpose. Moreover, they must cope with some body fluids like sweat or sebum. Additionally they must not emit toxic chemicals or chemicals which could interfere with the healing process of the body.

Adhesives to be used inside the body have to cope with an almost completely fluid environment, as the surfaces to be bound are immersed in blood and other body fluids. Applications of such adhesives have hitherto been proposed, but they are, on closer examination, scarce and restricted to very few systems such as alkyl-cyanoacrylates or fibrin.

Adhesives based on fibrin technology show a reasonable biocompatibility but low bond strength. This is prohibitive for their use in most load bearing applications, e.g. wounds where tension might occur. Additionally, they are very expensive since their biological origin requires extensive purification in order to avoid microbiological or allergenic contaminants.

Cyanoacrylate-based adhesives are reported to strongly interfere with the natural healing process. Moreover, there is toxicological concern with respect to the degradation products of some of these formulations.

Other adhesives, e.g. based on polyurethanes or epoxy resins, show excessive heat formation upon curing which has negative effects on wound healing and might even lead to tissue necrosis.

Another drawback with the known systems is that the adhesives have to be applied over a large area of the tissues, such that the adhesive subsequently acts as a barrier hindering the growth of new tissue within the wound, thus considerably interfering with the healing process.

It is an objective of the present invention to avoid the disadvantages known from prior art and to provide for a wound filling material showing beneficial effects on wound healing. This objective is achieved with a wound closure material according to claim 1.

A wound closure material according to the present invention has a core of biodegradable material. At least one side, but preferably two sides of said core of biodegradable material are provided with at least one, but preferably a multitude of discrete spots of an adhesive.

Biodegradable material as understood herein means a material which is over time completely disintegrated and resorbed by the body. This disintegration may be due to hydrolysis, oxidation or enzymatic cleavage. It is understood that a biodegradable material and none of its degradation should not be toxic to the organism, even at higher concentrations which may occur at the site of application. Moreover, such a material should preferably also not be allergenic. Use of a biodegradable material has the advantage that the wound closure material does not need to be removed but will gradually be replaced by new tissue.

At least one side of the core of the wound closure material is provided with at least one, but preferably a multitude of discrete spots of an adhesive. Preferably, at least two opposing sides of the core are provided with the at least one, but preferably the multitude of discrete spots of an adhesive.

A spot as understood herein is a small area of finite dimensions, without any restriction as to its shape or position. Therefore, a multitude of discrete spots is understood as to mean several spots having no direct connection with each other.

Having at least one, but preferably a multitude of discrete spots of an adhesive on at least one side of the core of biodegradable material has the advantage that the adhesive does not cover the entire surface of the at least one side, therefore leaving space for cells to grow directly onto and/or into the surface of the core. This facilitates degradation of the core as well as the ingrowth of new tissue cells into it.

The adhesive used to apply the spots has to be biocompatible and non-toxic. Preferably the adhesive is also biodegradable. Most preferably the degradation rate of the adhesive is slower as the degradation rate of the biodegradable material used for the core.

Having adhesive on at least one side of the core allows attaching the wound closure material into a wound and/or between edges of a wound.

In a preferred embodiment of the present invention at least two opposing sides of the core of biodegradable material are provided with at least one, but preferably a multitude of discrete spots of an adhesive. This has the advantage that the material can be used to close a wound by first adhering a first edge of the wound to a first side of the core and then subsequently adhering a second edge of the wound to the opposite side of the core, thereby tightly closing the wound. It is understood that more than one or two sides of the core may be provided with the at least one or multitude of discrete adhesive spots.

In an alternative embodiment, the core of biodegradable material comprises an open cell structure. Open cells are understood to be voids within the material which are in fluid connection with each other. These cells may be for example pores, channels and the like. This has the advantage that the core may be moistened by body fluids and that tissue cells can grow into said pores, thus gradually replacing the material of the core with new tissue. This has considerable beneficial effect on the rate of wound healing.

With the more 'classical' wound closure techniques like sutures, clips and tapes applied over the wound, new tissue has to close a gap between the wound edges. Use of a wound closing material of the present invention does not only have the advantage of a secure physical connection between the edges, but the material serves as scaffolding for the new tissue. It may also serve the cells as artificial extracellular matrix they can adhere to.

Preferably, the core of biodegradable material comprises at least a polyurethane foam with open pores. Biodegradable polyurethane foams are known in the art. Especially suitable polyurethane foams to be used in the present invention are for example described in European patent application EP 09167043.0 which is incorporated herein by reference in its entirety. The pores of the foam preferably have a largest diameter in the range of between 100µm and 400µm, most preferably between 100µm and 250µm. Alternatively, the core may comprise a web of non-woven material, preferably a silica gel fibre fleece. As understood herein, non woven material is a fabric-like material made from long fibres, bonded together by chemical, mechanical, heat or solvent treatment. Biodegradable non-woven materials are known in the field. Especially preferred a fibre fleece made of silica gel is used. Such a fleece has a multitude of open cells and is slowly degraded in the body while showing sufficient strength to be used as wound closing material.

Generally, the biodegradation rate of the core material should be in the range of 1 to 4 weeks such as to serve for ingrowth of cells and formation of new tissue. The degradation rate should be chosen according on the tissue the wound closure material is used in and the condition of the wound.

The core of biodegradable material is preferably provided as an area-measured material, preferably as a pad, tape, strip or polygonal sheet, most preferably in the form of a rectangular or circular sheet. This offers the advantage that the wound closure can be used to stick edges of a wound together. Moreover, such shapes may easily be cut and adapted to any wound size and shape by medical personal. Especially preferred is a core in the form of a tape roll which may be cut in an appropriate length prior to use. Such tapes might be provided in different widths and thicknesses.

The discrete adhesive spots are preferably provided as lines, dots or in a polygonal or circular shape on the at least one side of the core. The adhesive may be provided as lines of different length and width on the at least one side of the core. Alternatively, the adhesive may be provided as dots. Such dots are generally of circular shape and may have different diameters. Alternatively, the spots may be provided in any circular or polygonal shape. This includes rectangular, trapezoidal, triangular, round and oval shapes of any suitable dimension. The spots may all be provided in the same shape on the at least one side. Alternatively, the spots may be provided in different shapes. All shapes provided on the at least one side may have the same dimension and/or surface area. Alternatively, the shapes may be of different dimensions and/or surface areas.

Preferably, the discrete spots are, in first approximation, of circular shape with a diameter between 0.5mm - 5mm, preferably 0.6mm-2.5mm. The number of spots per side is chosen such as to maximize the area uncoated by the adhesive while simultaneously providing enough bond strength to stabilize the closure of the wound, i.e. resist the external forces affecting the wound. This ensures that a maximum of unobstructed tissue is available for the growth of new cells.

The discrete spots may each have a surface between 0.2mm² and 20mm², preferably between 0.5mm² and 5mm². Spots with a surface in this area have sufficient adhesive strength while still being small enough such as not to cover too much of the surface.

Preferably, the discrete adhesive spots are evenly spread over the entire area of the at least one side of the core of biodegradable material. This allows for an optimal distribution of tensile stress over the entire surface of the core side. Moreover, this allows for an optimal adherence of the wound filling material to the wound edges, which has a beneficial effect on the ingrowth of cells into the core.

Further, at least 30%, preferably at least 60%, most preferably at least 90% of the at least one side of the core of biodegradable material is not covered by the discrete spots of adhesive. This allows optimal growth of cells onto and/or into the core of biodegradable material. If too much of the surface of the side is covered with the adhesive, tissue cells will be hindered to grow onto and/or into the core of biodegradable material. If a too small amount of the surface is covered with the adhesive, only pore adhesion of the wound closure material to the wound edges will be achieved.

Preferably the adhesive spots are provided on the at least one side of the core of biodegradable material in a regular pattern. As an example, all spots may be provided such as to be regularly spaced apart from their neighbouring spots. Alternatively, the spots may be provided such as to generally define a geometrical pattern on the side. Alternatively, the adhesive spots are provided on the at least one side of the core of biodegradable material in a random pattern.

The adhesive of the adhesive spots should have an adhesive strength of between 0.05 to 20 MPa, preferably of between 0.1 to 10 MPa. The typical tensile strength of connective tissue, for example skin, is in the range of 0.01 to 8 MPa. It has to be taken into consideration that the spotty pattern of the adhesive reduces the total adhesive force by the ratio between the total surface and the sum of the surfaces of the spots within the surface.

Preferably the adhesive comprises a moisture curing polyurethane. The moisture curing polyurethane is preferably composed of an isocyanate-terminated polyesterpolyol pre-polymer or an isocyanate-terminated polyetherpolyol pre-polymer. Further the adhesive may also comprise a pressure sensitive adhesive. Preferably, the pressure sensitive adhesive has an increased stick in wet environments. Such adhesive are known in the art. The US2006/211808 exemplary describes a composition for pressure sensitive adhesives with an improved wet stick. Further, the adhesive may be based on polysaccharides such as alginates, pectins, polysaccharide gums, and other similar systems as well as on polymers which are activated by water such as gum arabic, polyvinyl pyrrolidone, polyvinylalcohol and other similar systems.

Alternatively, the adhesive comprises at least an adhesive from a biological source such as aquatic animals, like mussels and/or amphibia. Biological adhesives are mostly peptide based adhesive materials adapted to provide good adherence of biological tissue to other materials. Especially preferred thereby are biological adhesives from aquatic animals, since their adhesives are especially well adapted to a fluid environment. Alternatively or additionally, the adhesive may also comprise adhesive proteins from micro organisms, so called adhesins. Adhesins are used by micro organisms to attach themselves to host cells and therefore they show excellent adhesion qualities in an environment such as a wound. Alternatively, more 'classical' biological adhesives such as fibrin may be used. One advantage of the wound closure material of the present invention is that the amount of adhesive needed is considerably reduced by the application of the adhesive as spots. This leads to a reduction of costs for the material, since production and/or isolation of biological adhesives tends to be rather expensive.

Alternatively, hybrid adhesives consisting of a synthetic backbone, e.g. an acrylate, and biomimetic adhesives sites, e.g. polypeptides, may also be used in place of the pure biological materials.

Preferably the at least one side of the core of biodegradable material provided with the at least one spot of an adhesive is protected from the environment before use, preferably by a release liner. Especially in the case where there is the risk of premature curing of the adhesive when in contact with the atmosphere, for example when moisture curing polyurethanes are used, the side of the core bearing adhesive spots has to be protected. Use of a release liner offers the advantage that the protection can sequentially be removed from the wound closure material thus keeping adhesive spots on other areas than the one being used safe. Alternatively, the wound closure material may also be protected from the environment by means of a protective case, pouch and the like.

Another object of the present invention is to provide a method for closing a wound. The method comprises the steps of:
(a) applying a wound closure material according to the present invention to a wound in such a way that the at least two opposing sides provided with the at least one discrete spot of an adhesive are facing at least two wound edges;
(b) pressing the at least two wound edges together with the wound closure material in between the edges; and
(c) allowing the adhesive to cure so that the wound edges attach to the wound closure material.

Since the wound closure material of the present invention is provided with adhesive spots, there is enough space for tissue cells to grow onto and/or into the wound closure material between the adhesive spots. This has an advantageous effect on wound healing compared to cases where the adhesive is applied onto the entire surface of the wound. Moreover, compared to 'classical' wound closure techniques using sutures or the like, the wound closure material of the present invention serves as scaffold for new tissue cells. The wound closure material of the present invention may also act as artifical extracellular matrix for new tissue cells.

A further objective of the present invention is to provide a method of production of a wound closure material having improved wound healing qualities. The production method comprises the steps of:
(a) providing a core of a biodegradable material having an open cell structure
(b) applying a multitude of spots of an adhesive to at least one side of the core of biodegradable material, preferably by screen printing.

The method of production may further comprise the step of applying a removable protective sheet, preferably a release liner on the at least one side of the core provided with the adhesive spots.

Preferably the spots are applied by screen printing technique in step (b). Other techniques known in the art may also be used to apply the spots of adhesive to the wound closure material.

Another objective of the present invention is to provide a method of use of a wound filler material according to the present invention to close a wound. As mentioned, the wound closure material of the present invention provides improved wound healing qualities since the core material may serve as scaffolding and/or artificial extracellular matrix for new tissue cells. Moreover, since the adhesive is provided as discrete spots there is enough space between the adhesive for tissue cells to grow onto and/or into the core material without being hindered by the adhesive material.

In a preferred embodiment the wound closure material of the present invention is used to close a surgical transection. Compared to the 'classical' wound closure techniques, the wound closure material of the present invention provides an easy to use and quick way of closing transections. Moreover, the wound closure material of the present invention offers improved wound healing properties compared to other wound closure techniques.

Further advantages and characteristics of the present invention are described in the following description of examples and fig ures.
Fig. 1: An exemplary embodiment of a wound closure material according to the present invention.
Fig. 2: Three exemplary embodiments of different forms in which a wound closure material according to the present invention may be provided.

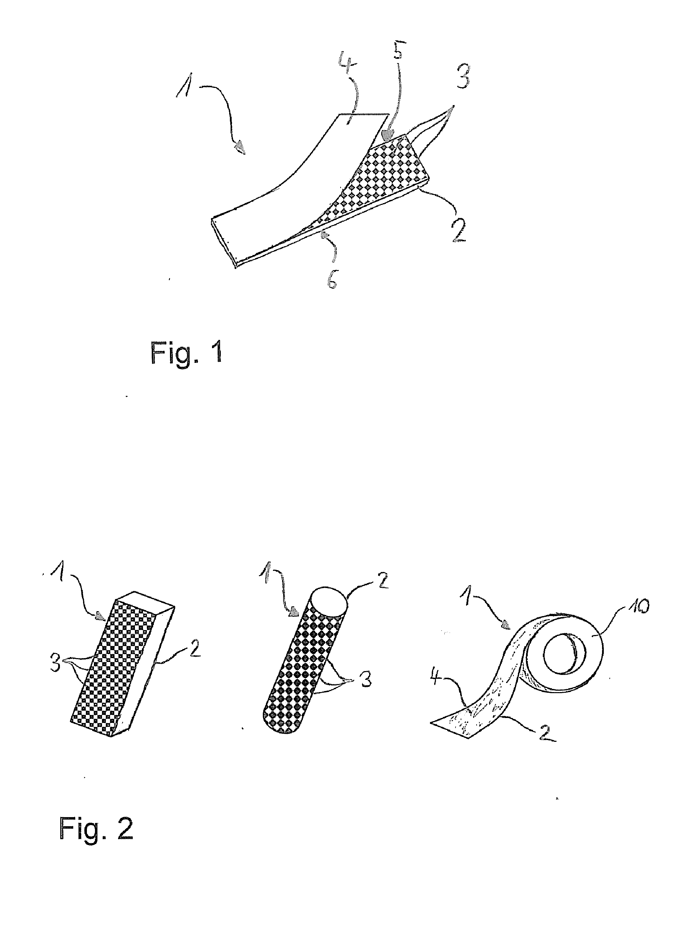

Figure 1 shows an exemplary embodiment of the wound closure material 1 of the present invention. Wound closure material 1 comprises a core 2 of biodegradable material with an open cell structure. Core 2 is shown as rectangular sheet. It is understood that core 2 might also be in any suitable form, like pads, tapes and the like. The core 2 preferably comprises a biodegradable polyurethane foam with an open pore structure or a biodegradable non-woven fleece material. A first side 5 of the core 2 is provided with a multitude of spots 3 of an adhesive. The spots 3 are shown as a pattern of rectangles distributed on the entire surface of the first side 5. Spots 3 might also be provided in any suitable geometric form, such as dots, circles, triangles and the like. The spots 3 might also be distributed in another pattern or randomly. A second side 6 opposing the first side 5 is preferably also provided with spots 3 of an adhesive.

In such a case the wound closure material can be used to close the edges of a wound together. Release liner 4 is provided on the first side 5 to protect spots 3 from the environment, thus hindering premature curing of the adhesive, e.g. due to moisture, and/or protecting the wound closure material 1 of adhering to any objects.

Figure 2 exemplarily shows three additional forms in which a wound closure material of the present invention may be provided.

Besides the sheet form shown in figure 1, the core 2 of wound cover material 1 may also be provided as rectangular or cylindrical pad. In the case of a cylindrical pad, the spots 3 of adhesive are preferably provided on the lateral surface. A third alternative is to provide the core 2 in the form of a tape, preferably as roll 10. In this embodiment, the core 2 is protected with release liner 4 to prevent sticking together of the core 2 when rolled up. The tape is preferably provided with spots 3 of adhesive on both sides.

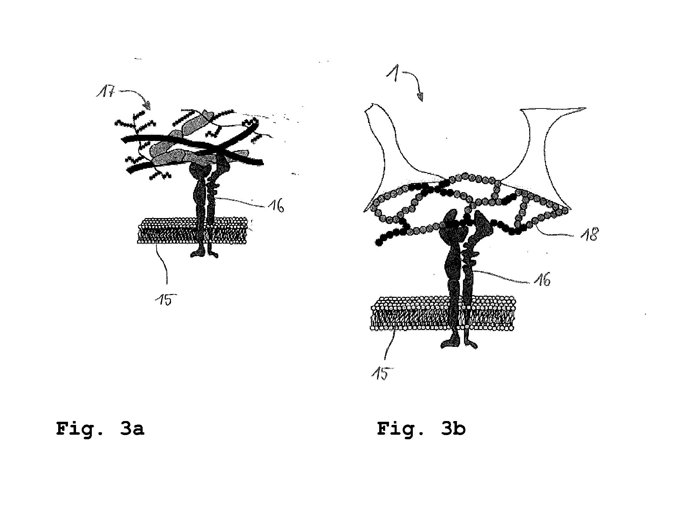

Figure 3a shows the undisturbed situation in which the plasma membrane 15 is connected to the extracellular matrix 17 by means of so-called adhesion molecules 16, especially integrins. Figure 3b schematically represents the wound closure material 1 on a molecular level. The adhesion molecules 16, specifically integrins, adhere to the adhesive polymer chains 18.

## Claims

1. Wound closure material with a core of biodegradable material, wherein at least one side, preferably two sides of the core of biodegradable material are provided with at least one, preferably a multitude of discrete spots of an adhesive.

2. Wound closure material according to claim 1, wherein at least two opposing sides of the core of biodegradable material are provided with at least one, preferably a multitude of discrete spots of an adhesive.

3. Wound closure material according to any of claims 1 to 2, wherein the core of biodegradable material comprises an open cell structure.

4. Wound closure material according to claim 3, wherein the core of biodegradable material comprises at least a polyurethane foam with open pores and/or a web of non-woven material, preferably a silica gel fibre fleece.

5. Wound closure material according to any of claims 1 to 4, wherein the core of biodegradable material is provided as an area-measured material, preferably as a pad, tape, strip or polygonal sheet, most preferably in the form of a rectangular or circular sheet.

6. Wound closure material according to any of claims 1 to 5, wherein the discrete adhesive spots are provided as lines, dots or in a polygonal or circular shape.

7. Wound closure material according to any of claims 1 to 6, wherein the discrete spots have a dimension of 0.5mm - 5 mm, preferably 0.6mm-2.5 mm.

8. Wound closure material according to any of claims 1 to 7, wherein the discrete spots each have a surface between 0.2mm² and 20mm², preferably between 0.5mm² and 5mm².

9. Wound closure material according to any of claims 1 to 8, wherein the discrete adhesive spots are evenly spread over the entire area of the at least one side of the core of biodegradable material.

10. Wound closure material according to claim 9, wherein at least 30%, preferably at least 60%, most preferably at least 90% of the at least one side of the core of biodegradable material is not covered by the discrete spots of adhesive.

11. Wound closure material according to any of claims 1 to 10, wherein the adhesive of the adhesive spots has an adhesive strength to human tissue of between 0.04 to 6 MPa, preferably of between 0.1 to 1 MPa.

12. Wound closure material according to any of claims 1 to 11, wherein the adhesive comprises a moisture curing polyurethane, preferably composed of an isocyanate-terminated polyesterpolyol pre-polymer or an isocyanate-terminated polyetherpolyol pre-polymer; a pressure sensitive adhesive; an adhesive based on polysaccharides; and/or an adhesive based on polymers which are activated by water.

13. Wound closure material according to any of claims 1 to 11, wherein the adhesive comprises at least an adhesive from a biological source such as mussels, amphibia or micro organisms, most preferably in the form of adhesins, and/or hybrid adhesives consisting of a synthetic backbone, e.g. an acrylate, and biomimetic adhesives sites, e.g. polypeptides, may also be used in place of the pure biological materials

14. Wound closure material according to any of claims 1 to 13, wherein the at least one side of the core of biodegradable material provided with the at least one spot of an adhesive is protected from the environment before use, preferably by a release liner.

15. Method of production of a wound closure material comprising the steps of:
(a) providing a core of a biodegradable material having an open cell structure;
(b) applying a multitude of spots of an adhesive to at least one side of the core of biodegradable material, preferably by screen printing.
